**Europäisches Patentamt**

**European Patent Office**

**Office européen des brevets**

(19)

(11) Veröffentlichungsnummer : **0 383 873 B1**

## (12) EUROPÄISCHE PATENTSCHRIFT

(45) Veröffentlichungstag der Patentschrift :
**10.03.93 Patentblatt 93/10**

(51) Int. Cl.$^5$ : **A61K 6/083**

(21) Anmeldenummer : **89908405.7**

(22) Anmeldetag : **21.07.89**

(86) Internationale Anmeldenummer :
**PCT/EP89/00856**

(87) Internationale Veröffentlichungsnummer :
**WO 90/00893 08.02.90 Gazette 90/04**

(54) **PULVERFÖRMIGER DENTALWERKSTOFF, VERFAHREN ZU SEINER HERSTELLUNG UND SEINE VERWENDUNG.**

(30) Priorität : **22.07.88 DE 3825027**

(43) Veröffentlichungstag der Anmeldung :
**29.08.90 Patentblatt 90/35**

(45) Bekanntmachung des Hinweises auf die Patenterteilung :
**10.03.93 Patentblatt 93/10**

(84) Benannte Vertragsstaaten :
**AT CH DE FR GB IT LI**

(56) Entgegenhaltungen :
**EP-A- 0 219 058**
**DE-A- 3 042 008**
**DE-A- 3 248 357**
**GB-A- 2 028 855**

(73) Patentinhaber : **THERA Patent GmbH & Co. KG**
**Gesellschaft für industrielle Schutzrechte**
**Am Griesberg 2**
**W-8031 Seefeld 1 (DE)**

(72) Erfinder : **GUGGENBERGER, Rainer**
**Inninger Strasse 13a**
**W-8031 Seefeld 2 (DE)**
Erfinder : **ELLRICH, Klaus**
**Auinger Strasse 16**
**W-8031 Wörthsee (DE)**
Erfinder : **GASSER, Oswald**
**Höhenstrasse 10**
**W-8031 Seefeld (DE)**
Erfinder : **McLEAN, John, W.**
**38 Devonshire Street**
**London W1N 1LD (GB)**
Erfinder : **MAKINSON, Owen**
**The University of Adelaide Dep. of Dentistry**
**Box 498, G.P.O. Adelaide, S.A. 5001 (AU)**

(74) Vertreter : **Abitz, Walter, Dr.-Ing. et al**
**Abitz, Morf, Gritschneder, Freiherr von**
**Wittgenstein Postfach 86 01 09**
**W-8000 München 86 (DE)**

EP 0 383 873 B1

## Beschreibung

Die Erfindung betrifft einen pulverförmigen Dentalwerkstoff nach dem Oberbegriff des Patentanspruches 1, dessen Herstellung und Verwendung.

Glasionomerzemente, die das Reaktionsprodukt aus einem Calciumaluminiumfluorosilicatglaspulver und/ oder einem Strontiumaluminiumfluorosilicatglas, einer wasserlöslichen Polycarbonsäure und Wasser darstellen, haben gegenüber anderen für Füllungszwecke verwendeten Dentalmaterialien (z.B. Composit-Kunststofffüllungen, Amalgam) den Vorteil, dass sie physiologisch sehr verträglich sind und mit der Zahnsubstanz eine chemische Verbindung eingehen, die einen perfekten Randschluss bewirkt.

Metallhaltige Glasionomerzement-Pulver sind aus der GB-A-2 028 855 bekannt. Bei diesem Material handelt es sich aber um ein blosses Gemisch von Zementpulver und Metallpulver, das keiner thermischen Behandlung unterworfen worden ist.

Aus der DE-A-32 48 357 ist ein pulverförmiger Dentalwerkstoff bestehend aus einer gesinterten Mischung aus Calciumaluminiumfluorosilicatglas und für dentale Zwecke üblichen Edelmetallen oder ihren Legierungen mit bis zu 40 Gew.-% unedlen Metallen bekann:. Dieser gesinterte Dentalwerkstoff besitzt eine deutlich höhere Verschleissfestigkeit als die Glasionomerzemente aus ungesintertem Dentalwerkstoff.

Aus der EP-A-0 219 058 sind polymerisierbare Zementmischungen bekannt, die (a) polymerisierbare ungesättigte Monomere und/oder Oligomere und/oder Prepolymere, die Säuregruppen und/oder deren reaktive Säurederivatgruppen enthalten, (b) reaktive Füllstoffe, die gegebenenfalls Sinterprodukte zwischen Gläsern und Edelmetallen sind und (c) Härtungsmittel enthalten. Die ungesättigte Verbindung wird durch das Härtungsmittel in Gegenwart des reaktiven Füllstoffes polymerisiert.

Aufgabe der Erfindung ist die Bereitstellung eines gesinterten, metallhaltigen Glasionomerzement-Pulvers, das bei vergleichbarer Qualität wie der Werkstoff der DE-A-32 48 357 ohne die Verwendung der teuren Edelmetalle herstellbar ist.

Diese Aufgabe wird erfindungsgemäss durch die Schaffung eines pulverförmigen Dentalwerkstoffes gemäss dem Oberbegriff des Patentanspruches 1 gelöst, der mindestens einen Teil des Bestandteils (a) als gesinterte Mischung mit dem Bestandteil (b) enthält.

Der neue pulverförmige Dentaluerkstoff wird erfindungsgemäss nach den Verfahren der Patentansprüche 2 oder 3 hergestellt. Das erhaltene gesinterte Produkt kann gegebenenfalls mit (1) nicht gesintertem Aluminiumfluorosilicatglas und/oder (2) mit trockener Polycarbonsäure und/oder (3) mit einem chelatbildenden Mittel, jeweils in pulverförmiger Form, vermischt werden.

Für die Verwendung wird der erfindungsgemässe pulverförmige Dentalwerkstoff im Falle (1) mit wässriger Polycarbonsäurelösung, die gegebenenfalls einen Chelatbildner enthält, im Falle (2) mit gegebenenfalls chelatbildnerhaltigem Wasser, z.B. einer Weinsäurelösung, und im Falle (3) mit wässriger Polycarbonsäurelösung oder mit Wasser zu einem Zahnfüllmaterial vermischt, das sich durch hohe Verschleissfestigkeit auszeichnet und daher auch zur Modellierung von Kanten und Ecken im Backenzahnbereich geeignet ist. Dabei haftet der aus dem erfindungsgemässen Dentalwerkstoff hergestellte Zement an der Zahnsubstanz und hat eine gute physiologische Verträglichkeit.

Als Glaspulver kommen neben den in der DE-A-20 61 513 und der DE-A-32 48 357 genannten calcium-, magnesium- und lanthanhaltigen Glaspulvern sowie strontiumhaltigen Glaspulvern der EP-A-241 277 auch Glaspulver mit anderen Kationen zum Einsatz. Bevorzugt verwendet werden Calcium- und/oder Strontiumfluorosilicatgläser, so dass die Aluminiumfluorosilicatglaspulver neben Sauerstoff folgende Bestandteile aufweisen können:

| Bestandteil | Berechnet als | Gew.-% |
|---|---|---|
| Si | $SiO_2$ | 20 – 60 |
| Al | $Al_2O_3$ | 10 – 50 |
| Ca | $CaO$ | 0 – 40 |
| Sr | $SrO$ | 0 – 40 |
| F | $F$ | 1 – 40 |
| Na | $Na_2O$ | 0 – 10 |
| P | $P_2O_5$ | 0 – 10 |

2

wobei mindestens 1 Gew.-% CaO und/oder SrO enthalten sein müssen und insgesamt 0 - 20 Gew.-%, berechnet als Oxide, an B, Bi, Zn, Mg, Sn, Ti, Zr, La oder anderen 3-wertigen Lanthanoiden, K, W, Ge sowie weiteren Zusätzen, welche die Eigenschaften nicht beeinträchtigen und physiologisch unbedenklich sind. Durch Zusatz von 10 - 20 Gew.-% $La_2O_3$ können die Gläser röntgensichtbar gemacht werden.

Vorteilhaft bestehen die Pulverteilchen aus

```
Si   als   SiO2          25 - 50 Gew.-%

Al   als   Al2O3         10 - 40 Gew.-%

Ca   als   CaO            0 - 35 Gew.-%

Sr   als   SrO            0 - 35 Gew.-%

F                         5 - 30 Gew.-%

Na   als   Na2O           0 -  8 Gew.-%

P    als   P2O5           1 - 10 Gew.-%
```

wobei mindestens 10 Gew.-% Ca (berechnet als CaO) und/oder Sr (berechnet als SrO) enthalten sein müssen und 0 - 10 Gew.-% an $B_2O_3$, $Bi_2O_3$, ZnO, MgO, $SnO_2$, $TiO_2$, ZrO, $La_2O_3$ oder anderen Oxiden 3-wertiger Lanthanoide, $K_2O$, $WO_3$, $GeO_2$ sowie weiteren Zusätzen, welche die Eigenschaften nicht beeinträchtigen und physiologisch unbedenklich sind.

Besonders bevorzugt verwendete Pulver enthalten

```
Si   als   SiO2          25 - 45 Gew.-%

Al   als   Al2O3         20 - 40 Gew.-%

Ca   als   CaO           10 - 30 Gew.-%

F                        10 - 30 Gew.-%

Na   als   Na2O           1 -  8 Gew.-%

P    als   P2O5           1 - 10 Gew.-%
```

Beispiele der bevorzugt verwendeten Zusammensetzungen sind in der folgenden Tabelle I aufgeführt:

## Tabelle I

Gew.-%

|  | A | B | C | D |
|---|---|---|---|---|
| Si als $SiO_2$ | 35,0 | 27,6 | 29,0 | 45,4 |
| Al als $Al_2O_3$ | 30,4 | 26,0 | 25,1 | 35,0 |
| Ca als CaO | 14,9 | 28,8 | 24,6 | 10,1 |
| F | 17,7 | 17,0 | 23,0 | 10,4 |
| Na als $Na_2O$ | 2,7 | 2,1 | 2,2 | 6,9 |
| P als $P_2O_5$ | 6,9 | 8,3 | 5,8 | 2,4 |

Die erfindungsgemäss verwendeten Glaspulverpartikel können an der Oberfläche an Calcium oder Strontium verarmt sein, wie es für Calcium in der EP-A-23 013 beschrieben ist. Insbesondere sind diese Pulver als Zumischung zum gemahlenen, metallhaltigen Sinterprodukt zu verwenden.

Die erfindungsgemäss verwendeten Glaspulver weisen eine durchschnittliche Korngrösse (Gewichtsmittel) von wenigstens 1 µm und bevorzugt mindestens 3 µm auf. Die durchschnittliche Korngrösse (Gewichtsmittel) beträgt 1-20 µm, bevorzugt 3-15 µm und besonders bevorzugt 3-10 µm. Die Teilchen haben eine maximale Korngrösse von 150 µm, vorzugsweise 100 µm, besonders 60 µm. Für die Verwendung als dentaler Befestigungszement beträgt die maximale Teilchengrösse 25 µm, vorzugsweise 20 µm. Zur Erzielung guter mechanischer Eigenschaften ist in üblicher Weise eine nicht zu enge Korngrössenverteilung günstig, wie sie beispielsweise durch übliche Vermahlung und Absiebung der Grobanteile erreicht wird.

Die Glaspulver werden wie üblich durch Zusammenschmelzen der Ausgangs-Komponenten bei Temperaturen oberhalb 950°C, Abschrecken und Mahlen gewonnen.

Als Metallpulver kommen alle, bei Raumtemperatur festen, bei Umgebungstemperatur praktisch nicht mit Wasser oder mit Luftsauerstoff reagierenden und physiologisch verträglichen Metalle in Frage. Insbesondere sind die Metalle Zinn, Titan, Tantal, Germanium, Niob, Aluminium und Zink zu nennen. Besonders bevorzugt sind Zinn, Niob, Titan und Tantal. Ganz besonders bevorzugt ist Zinn. Aber auch die Legierungen dieser Metalle untereinander kommen für den erfindungsgemässen Einsatz in Frage.

Die durchschnittliche Korngrösse (Gewichtsmittel) der Metallpulverkomponente (b) beträgt wenigstens 0,5 µm, bevorzugt wenigstens 1 µm und besonders bevorzugt mindestens 3 µm. Das Gewichtsmittel der Korngrössenverteilung liegt vorzugsweise zwischen 0,5 und 20 µm, insbesondere zwischen 1 und 10 µm. Die maximale Korngrösse der Metallpulver soll 60 µm, vorzugsweise 40 µm und ganz besonders 10 µm nicht überschreiten. Vorzugsweise weisen mindestens 90 % der Teilchen der Metallpulverkomponente (b) eine Korngrösse unterhalb 32 µm auf, besonders bevorzugt unterhalb 10 µm.

Der erfindungsgemässe pulverförmige Dentalwerkstoff kann auch trockene Polycarbonsäure enthalten; als solche können die zur Herstellung von Glasionomerzementpulver bekannten Polycarbonsäuren verwendet werden, z.B. Polymaleinsäure, Polyacrylsäure sowie Gemische davon, oder Copolymere, insbesondere Maleinsäure-Acrylsäure-Copolymere und/oder Acrylsäure-Itaconsäure-Copolymere.

Der erfindungsgemässe Dentalwerkstoff kann ausserdem ein chelatbildendes Mittel enthalten (vgl. DE-A-23 19 715). Vorzugsweise wird als Chelatbildner Weinsäure eingesetzt.

Zur Durchführung des erfindungsgemässen Verfahrens werden zunächst die pulverförmige Glaskomponente und die pulverförmige Metallkomponente miteinander vermischt. Der Anteil der Metallkomponente an der Gesamtmischung kann 20 - 80, vorzugsweise 40 - 60 Vol.-%, betragen.

Diese Mischung kann direkt - gegebenenfalls unter Schutzgas oder im Vakuum - gesintert werden; die Sintertemperaturen betragen > 150°C, vorzugsweise > 500°C. Die Mischung kann aber auch zunächst zu Formkörpern verpresst werden, die dann - gegebenenfalls unter Schutzgas oder im Vakuum - gesintert werden. Die Sinterung der Mischung oder der Formkörper wird vorzugsweise unter Druck - gegebenenfalls unter Schutzgas oder im Vakuum -,insbesondere oberhalb 50 MPa, durchgeführt. Insbesondere Mischungen oder Formkörper, die die niedrigschmelzenden Metalle Zinn und Zink enthalten werden bei Drücken oberhalb 50 MPa gesintert. Zu diesem Zweck werden Mischungen oder Formkörper und Pressform zunächst auf die gewünschte Temperatur vorgeheizt und anschliessend der Druck - gegebenenfalls unter Schutzgas oder im Vakuum - aufgebracht. Auf diese Weise genügen bereits Sinterzeiten von einigen Minuten in der Presse, um Sinterprodukte herzustellen. Auch Kombinationen der genannten Methoden sind möglich.

Das erhaltene Sinterprodukt wird fein vermahlen und anschliessend auf die gewünschte Korngrösse abgesiebt. Dabei werden die gleichen Korngrössen angestrebt, wie sie für die eingesetzten Aluminiumfluorosilicatglaspulver beschrieben wurden.

Zur Einstellung der Reaktivität kann das so erhaltene Pulver mit ungesintertem, dem oben beschriebenen Aluminiumfluorosilicatglas vermischt werden. Dabei sind Zusätze von 10 - 50 Vol.-% ungesintertes Glaspulver - bezogen auf das gesinterte Produkt - geeignet. Insbesondere kommen hier auch an der Oberfläche an Calcium oder Strontium verarmte Pulver zum Einsatz (vgl. europäische Patentschrift 23 013).

Man kann das gemahlene Sinterprodukt oder dessen Gemisch mit ungesintertem Glaspulver auch mit trockener, feinteiliger Polycarbonsäure im Gewichtsverhältnis 1:1 bis 5:1 vermischen.

Auch ein Zusatz von Pigmenten zur Verbesserung der Färbung kann vorteilhaft sein. Eine weitere Verbesserung der Farbe kann durch erneute thermische Behandlung des gemahlenen Sinterprodukts, gegebenenfalls nach Zusatz eines Pigments, erfolgen. Dabei wird vorteilhaft unter Schutzgas oder im Vakuum gearbeitet.

Man kann einem dieser Gemische auch ein chelatbildendes Mittel in Pulverform zusetzen.

Die erfindungsgemässen pulverförmigen Dentalwerkstoffe werden in der für Glasionomerzementpulver üblichen Weise verwendet. Falls der erfindungsgemässe Dentalwerkstoff keine trockene Polycarbonsäure enthält, so wird er im Gewichtsverhältnis 2:1 bis 10:1, vorzugsweise 4:1 bis 8:1, mit einer wässrigen Polycarbonsäurelösung vermischt, in der gegebenenfalls ein Chelatbildner, z.B. Weinsäure, gelöst ist. Es sind die gleichen Polycarbonsäuren in Form ihrer wässrigen Lösung geeignet, die in feinteiliger trockener Form mit dem erfindungsgemässen, metallhaltigen Pulver vermischt werden können. Enthält der erfindungsgemässe Dentalwerkstoff bereits trockene Polycarbonsäure, so wird der Dentalwerkstoff mit Wasser, gegebenenfalls unter Zusatz eines Chelatbildners, zu einer zementartigen Konsistenz angemischt.

Überraschenderweise werden mit den erfindungsgemässen Dentalwerkstoffen Zemente erhalten die unter Mundbedingungen gute Korrosionsfestigkeit aufweisen. Im allgemeinen können die in den erfindungsgemässen Werkstoffen verwendeten Nicht-Edelmetalle - insbesondere das Zinn - nicht ungeschütz dem Mundmilieu ausgesetzt werden, ohne dass in kurzer Zeit starke Korrosionserscheinungen auftreten.

Bei Einsatz der niedrigschmelzenden Metalle Zinn und Zink ist es ausserdem überraschend, dass die Gemische aus Glaspulver und Metallpulver bei Temperaturen oberhalb des Schmelzpunkts dieser Metalle gesintert werden können, ohne dass eine Separation des Glaspulvers vom (zusammengeschmolzenen) Metallpulver auftritt. Vielmehr erhält man nach dem Sintern bei diesen Temperaturen einen festen Sinterkörper mit gleichmässig verteiltem Metallanteil. Bereits bei Temperaturen weit unterhalb des Erweichungspunktes des Glases (oberflächliche Erweichung frühestens ab ca. 600°C feststellbar) erhält man ein festes Metall-Glas-Sinterprodukt.

Beispiel 1

Zinnhaltiges Sinterpulver

17,5 Gew.-teile eines Calciumaluminiumfluorosilicatglaspulvers der Zusammensetzung B der Tabelle I (durchschnittliche Teilchengrösse 6 μm) werden mit 82,5 Gew.-teilen eines feinteiligen Zinnpulvers (alle Teilchen 42 μm) homogen vermischt.

Die Mischung wird auf 200°C vorgeheizt und in einer ebenfalls auf 200°C vorgeheizten Form 3 Minuten lang bei einem Druck von 80 MPa zu zylindrischen Presslingen geformt, die einen Durchmesser von 40 mm und eine Höhe von 5 mm haben.

Das erhaltene feste Sinterprodukt wird fein vermahlen und < 63 μm gesiebt.

96 Gew.-teile dieses Sinterpulvers werden mit 4 Gew.-teilen Titandioxid homogen vermischt und 1 Stunde bei 500°C im Vakuum weiter thermisch behandelt.

64,5 Gew.-teile des metallhaltigen Sinterproduktes werden mit 35,5 Gew.-teilen des oben beschriebenen ungesinterten Calciumaluminiumfluorosilicatglaspulvers vermischt, das jedoch an der Oberfläche durch Behandlung mit 0,1 % Salzsäure, wie in der europäischen Patentschrift 23 013 beschrieben, verarmt wurde.

Der enthaltene Dentalwerkstoff wird im Gewichtsverhält nis 4,5 : 1 mit einer Lösung aus 37 g eines Copolymeren (1 : 1) aus Acrylsäure und Maleinsäure, 9 g Weinsäure und 54 g Wasser vermischt.

Die erhaltene Zementmischung hat eine Verarbeitungszeit von etwa 2,5 Minuten und eine Erhärtungszeit von etwa 5 Minuten.

Nach 24 Stunden (Lagerbedingung: 36°C, unter $H_2O$) hat der erhaltene Zement folgende physikalische Eigenschaften:

Druckfestigkeit: 195 MPa

Biegefestigkeit: 42 MPa

Oberflächenhärte: 380 MPa.

Die Korrosionsstabilität des erhaltenen zinnhaltigen Zements wurde mit dem silberhaltigen Zement nach Beispiel 2 der DE-A-32 48 357 verglichen:

1. Löslichkeit in Wasser nach ISO/DIS 7489 für Glasionomerzemente

zinnhaltiger Zement gemäss der Erfindung: 0,065 %

silberhaltiger Zement (DE-A-32 48 357): 0,05 %

2. Zylindrische Prüfkörper (Durchmesser 20 mm, Höhe 1,5 mm) wurden in je 50 ml Flüssigkeit 4 Wochen bei 36°C gelagert. Die Lösung wurde dann mittels Atomabsorptionsspektroskopie auf Zinn oder Silber untersucht.

| Medium | zinnhaltiger Zement (Sn-Konzentr. in ppm) | silberhaltiger Zement (Ag-Konzentration in ppm) |
|---|---|---|
| Wasser | 0 | 0 |
| Künstl. Speichel- lösung 1) | 3 | 1 |
| 0,05 n-Essig- säure | 7 | 2,4 |

1) Lösung II nach M. Rimpler et al. Deutsche zahnärztliche Zeitung 37, 322 (1982)

3. Analog zur ISO/DIS 7489 wurde die Löslichkeit in 0,05 n Essigsäure über einen Zeitraum von 4 Wochen bestimmt.

    zinnhaltiger Zement gemäss der Erfindung: 1,57 % lösliche Anteile

    silberhaltiger Zement (DE-A 32 48 357): 1,67 % lösliche Anteile

Im Vergleich mit dem silberhaltigen Zement ist das zinnhaltige, erfindungsgemässe Material nur geringfügig stärker (1., 2.) oder sogar etwas geringer (3.) korrodiert.

Beispiel 2

Titanhaltiges Sinterpulver

17,5 Gew.-teile eines Calciumaluminiumfluorosilicatglaspulvers der Zusammensetzung B der Tabelle I (durchschnittliche Teilchengrösse 6 μm) werden mit 82,5 Gew.-teilen eines feinteiligen Titanpulvers (alle Teil-chen < 40 μm) homogen vermischt. Dieses Gemisch wird 1 Stunde bei 1000°C gesintert und anschliessend vermahlen und < 63 μm abgesiebt.

87,5 Gew.-teile des vermahlenen Sinterprodukts werden mit 13,6 Gew.-teilen des ungesinterten Ausgangsglaspulvers vermischt.

Der erhaltene pulverförmige Werkstoff wird im Gewichtsverhältnis 3,5 : 1 mit der im Beispiel 1 beschriebenen, weinsäurehaltigen, wässrigen Polycarbonsäurelösung vermischt. Die erhaltene Zementmischung hat eine Verarbeitungszeit von ca. 3 Minuten und eine Erhärtungszeit von etwa 11 Minuten. Nach 24 Stunden (Lagerung bei 36°C, unter Wasser) zeigte der erhaltene Zement eine Druckfestigkeit von 171 MPa.

**Patentansprüche**

1.    Pulverförmiger Dentalwerkstoff auf der Grundlage von

     (a) Aluminiumfluorosilicatglas,

     (b) für eine dentale Verwendung geeigneten Nicht-Edelmetallen oder ihren Legierungen untereinander, ausgewählt aus der Gruppe Zinn, Titan, Tantal, Germanium, Niob, Aluminium und Zink, und gegebenenfalls

     (c) trockener Polycarbonsäure und/oder

(d) chelatbildendem Mittel,
dadurch gekennzeichnet, dass er mindestens einen Teil des Bestandteils (a) als gesinterte Mischung mit dem Bestandteil (b) enthält.

2. Verfahren zur Herstellung des pulverförmigen Dentalwerkstoffs des Anspruchs 1, dadurch gekennzeichnet, dass man Pulver von (a) Aluminiumfluorosilicatglas und (b) für eine dentale Verwendung geeigneten Nicht-Edelmetallen oder ihren Legierungen untereinander vermischt, das erhaltene Gemisch, gegebenenialls nach Verpressung zu Formkörpern, bei > 150°C sintert, das gesinterte Produkt zu einem Pulver vermahlt und gegebenenfalls das erhaltene Pulver mit (a) ungesintertem Aluminiumfluorosilicatglas, (c) trockener Polycarbonsäure und/oder (d) chelatbildendem Mittel, jeweils in pulverförmiger Form, vermischt und gegebenenfalls das Pulver und/oder das Gemisch erneut thermisch behandelt.

3. Verfahren nach Anspruch 2, dadurch gekennzeichnet, dass die Sinterung unter Druck erfolgt.

4. Verwendung des pulverförmigen Dentalwerkstoffs des Anspruchs 1 zur Herstellung von selbsthärtenden Glasionomerzementen.

## Claims

1. Dental material in powder form based on
   (a) aluminium fluorosilicate glass,
   (b) base metals suitable for a dental use or their alloys with each other, selected from the group tin, titanium, tantalum, germanium, niobium, aluminium and zinc, and optionally
   (c) dry polycarboxylic acid and/or
   (d) chelating agent,
   characterised in that it contains at least a part of constituent (a) as sintered mixture with constituent (b).

2. Process for the preparation of the dental material in powder form of claim 1, characterised in that powders of (a) aluminium fluorosilicate glass and (b) base metals suitable for a dental use or their alloys with each other are mixed, the mixture obtained is sintered at >150 °C, optionally after compression to moulded bodies, the sintered product is ground to a powder and, optionally, the powder obtained is mixed with (a) unsintered aluminium fluorosilicate glass, (c) dry polycarboxylic acid and/or (d) chelating agent, in powder form in each case and, optionally, the powder and/or the mixture is heat-treated again.

3. Process according to claim 2, characterised in that sintering takes place under pressure.

4. Use of the dental material in powder form of claim 1 for the preparation of self-setting glass ionomer cements.

## Revendications

1. Matériau dentaire pulvérulent à base de
   (a) verre de fluorosilicate d'aluminium,
   (b) métaux communs appropriés à l'application dentaire ou leurs alliages entre eux, choisis dans le groupe étain, titane, tantale, germanium, niobium, aluminium et zinc et le cas échéant
   (c) acide polycarboxylique sec et/ou
   (d) agent formateur de chélate,
   caractérisé en ce qu'il contient au moins une partie du composant (a) sous forme de mélange fritté avec le composant (b).

2. Procédé de préparation d'un matériau dentaire pulvérulent de la revendication 1, caractérisé en ce qu'on mélange une poudre de (a) verre de fluorosilicate d'aluminium et (b) métaux communs appropriés à l'utilisation dentaire ou leurs alliages, en ce qu'on fritte à plus de 150°C le mélange obtenu, éventuellement après compression pour donner un corps moulé, en ce qu'on broie le produit fritté pour obtenir une poudre et le cas échéant en ce qu'on mélange la poudre obtenue avec (a) un verre de fluorosilicate d'aluminium non-fritté, (c) un acide polycarboxylique sec et/ou (d) un agent formateur de chélate, à chaque fois sous forme pulvérulente, et le cas échéant en ce qu'on procède à nouveau à un traitement themique de la poudre et/ou du mélange.

3. Procédé selon la revendication 2, caractérisé en ce que le frittage s'effectue sous pression.

4. Application du matériau dentaire pulvérulent de la revendication 1 à la préparation de ciments d'ionomères de verre autodurcissants.